(19) Europäisches Patentamt
European Patent Office
Office européen des brevets

(11) **EP 4 544 977 A1**

(12) **EUROPEAN PATENT APPLICATION**

(43) Date of publication:
**30.04.2025 Bulletin 2025/18**

(21) Application number: 23205911.3

(22) Date of filing: **25.10.2023**

(51) International Patent Classification (IPC):
**A61B 1/00** (2006.01)    **A61B 1/045** (2006.01)
**G06T 7/571** (2017.01)    **H04N 13/271** (2018.01)
**H04N 23/50** (2023.01)    **A61B 1/04** (2006.01)
**A61B 34/30** (2016.01)    **G06T 7/00** (2017.01)

(52) Cooperative Patent Classification (CPC):
**A61B 1/00194; A61B 1/00009; A61B 1/045;**
**H04N 13/271;** A61B 1/00147; A61B 1/0019;
A61B 1/042; A61B 2034/301; H04N 23/555

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**KH MA MD TN**

(71) Applicant: **JOSHI INNOVATIONS GmbH**
**78573 Wurmlingen (DE)**

(72) Inventor: **Joshi, Shirish**
**78573 Wurmlingen (DE)**

(74) Representative: **Müller Schupfner & Partner**
**Patent- und Rechtsanwaltspartnerschaft mbB**
**(Muc)**
**Bavariaring 11**
**80336 München (DE)**

(54) **IMAGING DEVICE, ENDOSCOPE, SYSTEM AND METHOD FOR IMAGING AN OBJECT**

(57)    An imaging device (1) for generating imaging data of an object (2) is provided. The imaging device (1) comprises: an image controller (3) configured to control an image sensor (4) to acquire first image data of the object (2), having a first image setting, and second image data of the object (2), having a second image setting, wherein the first image setting and the second image setting differ from each other, wherein the image controller (3) is further configured to switch the image settings between the first image setting and the second image setting in less than 100ms, a data processor (5) configured to receive the first image data and the second image data and to forward the image data to a 3D engine (6), wherein the 3D engine (6) is configured to generate a depth map of the object (2) based on the image data. Further, an endoscope (10), a system and a method for imaging an object (2) is provided.

FIG. 1

EP 4 544 977 A1

# Description

The present invention relates to an imaging device, an endoscope, a system and a method for imaging an object.

Imaging devices may be used to acquire images of an object to be examined. The imaging device may be provided in a laparoscope or endoscope or microscope that is used as a videoscope, often also called videoscope or video probe. In such endoscopes an image sensor is attached to an objective of the endoscope or near the ocular and is configured to acquire an image of the object under examination; which may be an organ inside the body or another kind of object that is difficult to access, such as the inside of a machine. Therefore, endoscopes are slim devices with little space inside. Hence, it is difficult to include an imaging system inside the endoscope that may be used to acquire detailed images of an object to be imaged.

In addition, it becomes even more complicated if more information is required than only 2D imaging of the object under examination. In modern applications it is often desirable to implement a 3D reconstruction.

In the prior art there are known systems having a stereoscopic approach to achieve 3D reconstruction. For example, US 2013038689 A1 shows such stereoscopic approach.

However, due to minimalization, there is a need for smaller systems. In addition, higher accuracy is desired as the use of robots in handling such devices increases.

It is therefore an aim of the present invention to provide an apparatus and method capable of providing a real time 3D reconstruction with high accuracy and, at the same time, a smaller size, at lower cost and without hindering the working of user.

The present invention solves this problem with an imaging device including the features of claim 1, with an Endoscope including the features of claim 13, with a system including the features of claim 14 and with a method for imaging an object including the features of claim 15.

According to one aspect of the present invention there is provided an imaging device for generating imaging data of an object. The imaging device may comprise an image controller configured to control an image sensor to acquire first image data of the object, having first image setting, and second image data of the object, having second image setting, wherein the first image setting and the second image setting differ from each other, wherein the image controller is further configured to switch the image settings between the first image setting and the second image setting in less than 100ms. The imaging device may further comprise a data processor configured to receive the first image data and the second image data and to forward the image data to a 3D engine. The 3D engine may be configured to generate a depth map of the object based on image data.

In contrast to the available prior art, the present invention provides the image controller which is configured to cause the image sensor to acquire first and second image date having different image settings. Accordingly, the first image data may be used to depict the object. The first image setting for the first image data may be intended for optimum viewing by a user. For example, the first image data may be visualized to a user handling the imaging device or any other device which works in combination with the imaging device. The first image data may have a focus, sharpness and/or iris adjusted as per need of the user. The second image data may on the other hand be used to generate additional supplementary information to improve accuracy of a depth map. The second image data may not be directly depicted to the user. If depicted, it may be for very short time so that the user does not feel disturbed by it. In particular, depth information may be generated by using the first image data, but this is not of sufficient accuracy required in surgery. Especially when the whole image is in focus and there are no known objects included in the first image data(e.g., instruments, etc.). When the image data has varying degree of focus or slightly out of focus, more information can be obtained for depth estimation. However, such image data is suboptimal for the user. In general, different image settings may be required for the pure display of an image and for the determination of a depth map of the object. Since the image controller is configured to change the image setting in less than 100 ms, the normal visualization of the object (i.e. on the basis of the first image data) is not disturbed. In other words, the second image data (i.e. the image data responsible for the depth map) can be acquired during a normal work process where the user is guided through images representing the object (i.e. based on the first image data) without realizing that the image setting has been changed. In other words, the image setting is changed so quickly that the user does not notice it. This way, the device may be minimalized because there is no second optical path, prisms, a plurality of sensors etc. necessary to obtain both the first image data and the second image data. Both the first and second image data can be used for depth estimation along with respective settings. The image sensor and optical system are pre-calibrated to know focal plane distance, optical aberrations and distortions. Also, the speed of effecting the setting changes is known and their effect of image during this process.

In general, rotation of an endoscope is very challenging when a stereo endoscope is used for imaging. When such endoscope is rotated, both image channels rotate around the axis. Because human brain is only able to handle images when the two images are horizontal. Also, stereo optical systems are much more complex and expensive to produce. While stereo optics provide 3D visualization, they do not provide a depth map. Creating an accurate depth map from them is complex because of optical aberrations, etc.

According to an aspect of the present invention, a depth map is derived from degree of focus or defocus in the image data. When an object is out of focus, it may be either in front of the image plane or behind the image plane. This may be guessed from prior knowledge of the scene. In surgical situation, the prior knowledge may not be available because of indistinguishable objects or a dynamic scene. Also, for surgical use, the depth map generated needs to be accurate to less than few millimeters. Traditional optical systems for surgery are designed for high depth of focus. This limits the possibility to generate a depth map with required accuracy. Inaccuracy in depth map may put a patient at risk.

In general, a feed of image data (e.g., a video feed) acquired during surgeries consists of several frames per second. Frame rate of 50 or 60 may be used. The human eye is not able to differentiate of one or few of these frames are skipped or missed or slightly modified such has have lower resolution or different focus. The imaging device may quickly change image settings and the image sensor may acquire image data without knowledge of user or disturbing the workflow of user. Thus, the acquisition of second image data may happen in the background. As a result, the user is unaware of this process. The second image data may then be used to generate depth information using similar methods such as described in EP 4000494 A1 which is incorporated herein by reference. In more detail, the imaging device may allow to take multiple snapshots of the area under observation at different focus, for example. Such snapshots may be combined with live video feed to accurately determine a depth map. The image data obtained for determination of the depth may or may not be shown to the user. As a result, a more accurate 3D reconstruction of the area under observation (e.g., the object) may be realized without disturbing the user's workflow. This may enable the user to keep working as usual and yet have good accuracy of 3D reconstruction.

The imaging device may be an apparatus usable together with imaging systems. For example, the imaging device may be placed inside a camera head of an endoscope or at a tip of an endoscope. Further, a camera can be mounted on the imaging device. The imaging device may be also placed inside or on surgical microscope. The imaging device may be mounted independently on a microscope or mounted on a beam splitter to allow a user to see the image through the optics. When used on binocular microscope, the imaging device may be mounted on one or both optical paths. The imaging device may be applied in the area of surgery.

Especially, using the imaging device, surgeries done with minimal invasive techniques such as laparoscopy will be improved. However, surgeries may also be done using microscope or open surgery. The imaging device may also be used for open surgery. In such case an image sensor may be mounted on a holder and targeted at surgical site. The imaging device may also be very advantageously applied for robotized surgeries. The depth map (i.e., the 3D reconstruction) may help robotized arms move with greater precision and avoid unintended damages.

The image controller may be a means for controlling other devices or apparatuses. The image controller may be hardware or software or both. The image controller may be configured to output commands to the image sensor about how and when to capture the image data. The commands may be indicative of when image data is to be captured and with what image setting the image data is to be captured. In other words, the image controller may be output a command indicative when to acquire first image data and when to acquire second image data. In particular, the image controller may output a command indicative of the image setting with which the second image data is to be acquired. For example, how the focus should be changed with respect to the first image data. The command may further be indicative of a timing and/or the amount of image data to be acquired. The image controller may be in communication with the image sensor. The image controller may be configured to control optical and / or mechanical system (opto-mechanics) such as liquid lens, iris adjustment and/or piezo adjustment system.

The image sensor may be referred to as a photo sensor or sensor chips. Preferably, the minimum resolution the image sensor is 1080 x 1920 pixels. The image sensor may be a complementary metal-oxide-semiconductor (CMOS) sensor, also known as complementary-symmetry metal-oxide-semiconductor (COS-MOS) sensor. Further, the image sensor may be charge-coupled device (CCD) sensor. In case a CMOS sensor is used, the image data may be a voltage signal that is outputted by the image sensor. Preferably, each pixel of the image sensor may have a specific voltage signal. That is, CMOS sensor may output a digital signal. In case the CCD sensor is used, the image data may be a charge signal. The voltage signal is less prone to be deteriorated by electromagnetic fields and thus a CMOS sensor is preferably used as the image sensor. The image sensor may also be Indium gallium arsenide (InGaAs) for shortwave infrared imaging. Indium gallium arsenide (InGaAs) (alternatively gallium indium arsenide, GaInAs) is a ternary alloy (chemical compound) of indium arsenide (InAs) and gallium arsenide (GaAs).

The image data may be an output of the image sensor. Moreover, the image data may include brightness information for one or more color channels. A color channel may represent a specific light spectrum. For example, the image data may comprise information for the green color channel, the RGB color channel and/or the IR (infrared) color channel. The RGB color channel is considered as including the green color channel, the red color channel and the blue color channel. Further, the image sensor may provide image data including information of different color channels. For example, color channels of the image sensor may be NIR-NIR, RGB-RGB, green-green, NIR-RGB, green-NIR, green-RGB. Wherein each color channel may be defined by a different band of wavelengths. For further processing of the image data, the imaging device may include the data processor or may be connected or connectable to a processing unit (e.g. a computer) or to a control unit. The data processor may be configured to further process the image data. For example, the data processor may determine the information contained within the image data, e.g. a histogram showing the brightness distribution. The data processor may be configured to improve acquired image data by applying noise reduction and/or sharpness enhancing algorithms. It may also enhance the dynamic range of image data to make it better for analysis and/or viewing. In addition, the image data may comprise an image or photograph depicting the object. In order to cope with bad light conditions, the imaging device may have a light source configured to light the object to be imaged. For example, the imaging device may be connectable to an external light source and may be configured to control the light source. In more detail, the light source may be coupled with an additional wave guide that is configured to guide the light of the light source to the object to be imaged.

The image setting may be indicative for boundary conditions of the acquisition of image data. That is, the image setting may influence the image data. The image setting may be set by the image sensor and/or by other devices. The image setting may include an aperture, depth of focus, light, shutter speed and/or focal length. The image setting may be indicative for one or more of the above properties. The image data may be changed in accordance with the change of the image setting. A switch of the image setting may be realized when at least one condition defined by the image setting changes. In this way, the second image data may be used for improving depth map and/or 3D reconstruction. For example, when the first image data has a different focus (i.e., a different focal plane) as the second image data, the first image setting differs from the second image setting.

The process of switching between the first image setting and the second image setting may be a process of changing at least one of the boundary conditions being responsible for the image data. That is, the switching may be completed when from a first condition (e.g., in which the first image setting is implemented) the image settings are switched to a second condition (e.g., in which the second image setting is implemented). In the first and in the second condition image data may be acquired. The switchover can take place in less than 100 ms. By implementing the above switching of the image setting (e.g., the focus), the focus may be changed within less than 100 ms, one or more frames may be captured (e.g., the second image data) and focus may be changed back to original focus. All this may happen within less than 300 ms at most. This sequence of changing image setting, capturing image data, switching focus back to original image setting may be called as capture sequence B (e.g., second sequence). As all this Sequence B happens in very short time, the user can work without affecting his workflow. The image data which do not have the first image setting may be called image B (second image data). In one sequence B there may be image data with different second image setting (e.g., focal distance). The image data taken with first image setting may be called Sequence A (first sequence). For example, a liquid lens may change the image setting very fast (e.g., focal distance). Alternatively or additionally, a piezo drive may also change image settings very fast by acting very fast over short distances and maintain high accuracy. Accordingly, a piezo drive may be implemented to change the image setting. Such means may allow the target focus to be set in 20 ms (e.g. second image setting), the image data (second image data) may be captured in 20 ms and the focus may be reset to the original focus in 20 ms (e.g. the first image setting). Hence, the total process may preferably happen within 60 ms. Since this time span is very short, it will not be perceptible to the user, even if they are actively manipulating the tissue based on the first image data.

The switch between the first image setting and the second image setting may be executed in accordance with a timing. The timing may be synchronized so that the image setting and image data obtained correlates with each other. The image sensor may include a shutter. A shutter speed of the shutter may be fixed or variable. A known shutter helps in synchronization of image data and image setting.

The data processor may be a device being configured to receive data, process data and output data. For example, the data processor may be a CPU, GPU or FPGA or any other processing unit. The data processor may be configured to receive image data directly from the image sensor or through an image processor that may be configured to improve the image data for further analysis. The image data may be outputted by the image sensor and may be directly received by the data processor. The data processor then may process the image data. The processing may include recognition of what image data is first image data (image A) and what image data is second image data (image B). This may be based on the image setting, which may also be known to the data processor. The image setting may be separately inputted to the data processor and/or the data processor may determine the image setting based on the received image data. The processing may further include separating the first image data and the second image data. That is, the data processor may provide first image data and second image data as separate output data. In other words, the image data stream inputted to the data processor may be processed in two image data streams as an output. Accordingly, the first image data and the second image data may be forwarded to different subsequent processes (e.g., the image viewer and to the 3D engine). One of the subsequent processes may be realized by an image viewer. The image viewer can be configured to display the target area (e.g. the object) that the user wants to examine. The image viewer may be provided with the first image data.

Accordingly, the first image data captured with the first image setting may be advantageously used for visualizing the object to be examined. In other words, the image viewer may not be able to handle the second image data as the second image setting may not be suitable to sufficiently visualize the target area (e.g., due to a small focal depth, different focal plane). On the other hand, the other subsequent process may be realized by the 3D engine. The 3D engine may be configured to advantageously handle the second image data (image B). In addition, the 3D engine may be configured to also handle the first image data (image A). In more detail, the 3D engine may be configured to generate a depth map based on the second image data and the first image data. That is, the 3D engine may be configured to generate depth information of the object based on the first and second image data (i.e., a depth map of the object). The 3D engine may be a device similar to a computer and may be configured to receive input data, to process the input data and to output processed data. In particular, the image data (e.g. a plurality frames) including 2D coordinates of the object may be the input data and a third coordinate (i.e. the depth information or 3D shape information) of the object may be the output data. That is, the 3D engine may output a depth map (e.g. 3D image data) of the object. Specifically, the depth map may be a scatter plot of points each having three coordinates describing the spatial position or coordinate of each point of the object. That is, the 3D engine may be configured to determine a depth map based on the image data. The image data may comprise at least two frames (e.g., two images of the object). The at least two frames have different image settings. That is, the at least two frames are acquired with different image settings which differ from each other.

Further, the 3D engine may be configured to divide the first and second frames of the image data into segments of one or several (e.g. >9) pixels, referred to as patches. In addition, the 3D engine may be configured to compare a patch of the first frame of the image data with a patch of second frame of the image data. The patches may be rectangular or quadratic patches. In particular, the 3D engine may be configured to determine how sharp the object is depicted in each patch (i.e. to determine a sharpness of each patch). The patches may have multiple sizes. That is, the size of the patches provided in the respective frames may vary depending on the object that is depicted by the frames and/or on the specific application (e.g. the kind of surgery). That is, in areas where a lot of texture exists, the patch size may be smaller (e.g. 5 x 5 pixels), whereas in areas of the image data where pixel intensities are more homogeneous, i.e. there is little texture, the pixel size may be larger (e.g. 50 x 50 or up to 1000 pixels). For example, a lot of texture may be in an area of the frames where a lot of edges are depicted. Accordingly, the 3D engine may work in a highly efficient way, because in areas where there is a lot of texture, the patch size is small (i.e. the resolution is high) so as to obtain highly accurate information of this area, whereas in areas where there is little texture, the patch size is bigger so as to accelerate the process carried out by the 3D engine.

Preferably, the location of the at least one or each first patch in the first frame of the image data corresponds to the location of the at least one or each second patch in the second frame of the image data. Preferably, the at least one first patch preferably has the same size as the at least one second patch, preferably a size of 20 x 20 pixels. That is, the first patch has the same location within the first frame of the image data as the second patch within the second frame of the image data. In more detail, if the first frame of the image data and the second frame of the image data are registered with one another, the first patch and the second patch overlap with one another. Accordingly, for each patch a depth value (i.e. the z-coordinate) may be determined by the 3D engine (i.e. for each patch x, y and z coordinates in 3D space are determined). A patch may have a size of one or several pixels. However, the required computing resources are depending on the number of patches included within the frames. Therefore, preferably each patch may have a size of 20 x 20 pixels. Having this size, the efficiency of the system is ensured while a high accuracy of the depth map is ensured.

Further, the 3D engine may be configured to determine the sharpness of each patch of the first frame of the image data and each patch of the second frame of the image data. It is to be noted that several patches may have the same sharpness. The sharpness of each patch is proportional to the entropy of the respective patch. In other words, the sharpness is synonymous for the entropy. A patch of the first frame of the image data may be considered as a first patch and a patch of the second frame of the image data may be considered as the second patch. The first patch and the second patch may overlap perfectly with one another (i.e. depicting the same scene/part of the object). In other words, the first patch and the second patch (also referred to as a pair of patches) may or may not be located at the same location within the first frame of the image data and within the second frame of the data. As explained above the second image setting may be set by the image controller with objective of optimizing depth map. The image settings for first image data may also be set by image controller with objective of optimizing image for the user. Therefore, the image settings (e.g., focus distances) of the first frame of the image data and the second frame of the image data is known. Using the following formula the 3D engine may determine the depth (i.e. the z-coordinate) of the part of the object depicted by the first patch and the second patch.

$$d = \frac{(d_1 * I_1 + d_2 * I_2)}{(I_1 + I_2)}$$

wherein

d     is the unknown distance (i.e. depth or z-coordinate) of a part of the object depicted in the first patch and the second patch,

$d_1$     is the focus distance of the first frame of the image data,

$I_1$     is the sharpness of the first patch,

$d_2$     is the focus distance of the second frame of the image data,

$I_2$     is the sharpness of the second patch.

The focusing distances of the first frame of the image data and the second first frame of the image data and the patch size has to be selected carefully so as to attain useful depth information using the above formula. Preferably, the focus distances are dependent on the specific application that is executed (e.g. the specific surgery or laparoscopy that is performed). For example, the focus distance of the first frame of the image data may be 6 cm measured from a distal end of the shaft and the focus distance of the second frame of the image data may be 9 cm from the distal end of the shaft. The focus distance of the first frame of the image data may an empirical value determined from the type of surgery (e.g. for laparoscopy it may be 6 cm measured from the tip of the laparoscope (i.e. the distal end of the shaft)).

The sharpness of the image may be expressed by the information density of the frame (e.g. the entropy) of the specific patch. The 3D engine may be configured to apply the above formula to each pair of patches (i.e. to each first patch and to each corresponding second patch) of the first frame of the image data and the second frame of the image data. As a result, a depth value for each pair of patches of the image data may be determined by the 3D engine. Having the depth information, the 3D engine may be configured to create a depth map using the depth information and the x and y coordinates of the respective pair of patches. In other words, the 3D engine may be configured to create a 3D model based on the x, y and z coordinates of each pair of patches.

Alternatively or additionally, the 3D engine may be configured to create a 3D model using the method of focus stacking. A further method that may be used to create a depth map is the so-called depth from focus/defocus. In addition, the control unit may be configured to carry out one or more of the above methods in order to create the depth information (i.e. 3D image data) based on at least two 2D image data (i.e. first frame of the mage data and the second frame of the image data). Specifically, the 3D engine may be configured to combine at least two methods for creating a depth map. In particular, the above formula may be applied to patches of the image data having a relatively high texture compared with other patches of the image data. In addition, the shape from lightning method may be applied to other regions of the image data having relatively poor texture compared with other regions of the image data. As a result, the performance of depth map generation may be significantly improved by applying different methods to different portions of the image data. Hence, at least one embodiment of the present invention may convert a relative difference of image settings (e.g., focus) of the image data into 3D shape information. In other words, 3D coordinates of all patches within the image data may be calculated/determined by comparing the focus/blur of the respective patch (i.e. of one pixel or an array of pixels). Optionally, the 3D engine may be configured to further process the depth map by filtering the resulting depth map. In more detail, in order to delete erroneous depth information, the control unit may be configured to compare depth information of the depth map adjacent to each other. In case, one depth is excessively high or low (i.e. exceeding a preterminal threshold) compared to its neighbors, the 3D engine may be configured to delete such depth information because it is most likely that this depth information is an error. As a result, the outputted depth map by the 3D engine may have an increased accuracy.

Preferably, the 3D engine is further configured to generate the depth information by comparing the entropy of at least one first patch of the first frame of the image data and of at least one second patch of the second frame of the image data. The sharpness of the image is a synonym to the entropy. The entropy is an example of the information (i.e. a measure of the sharpness of a patch of the image data) included within the image data. That is, the first frame of the image data and the second frame of the image data may be compared to each other on the basis of their entropy. Accordingly, the 3D engine may be configured to determine the entropy of the first frame of the image data and of the second frame of the image data. Specifically, the higher the entropy, the more the respective image data is in focus. Accordingly, the lower the entropy, the less the respective image data is in focus (i.e. is blurred). The entropy may be determined by the following formula:

$$H = -\sum_{k} p_k \log_2(p_k)$$

wherein k is the number of levels of one channel or band (e.g. the gray-channel or the green-channel under observation) and $p_k$ is the probability associated with gray level k. Further, as the band the RGB-channel and/or the IR-channel may be used. The sharpness $I_1$ and $I_2$ used in the above formula for determining the depth of a specific part of the object, may be respectively substituted by the entropy. That is, using the above formula, the entropy $H_1$ is determined for the first patch and the entropy $H_2$ is determined for the second patch (and for each further pair of patches). Then, $H_1$ and $H_2$ are used instead of the $I_1$ and $I_2$ in the formula for determining the depth of a specific part of the object depicted in the first patch and the second patch. That is, the entropy is synonymous for the sharpness.

According to the present invention at least two different image data (i.e., first image data and second image data) may be received or generated. Each image data may be suitable for different subsequent processes. The second image data may be suitable for being used in determination of a depth map (e.g., by a 3D engine). The second image data may comprise at least one frame. In case more than one frame is included in the second image data each frame of the second image data may have different second image settings. Accordingly, some portions of the object may be in focus on the first frame of the second image data, while some other portions of the object may be in focus on the second frame of the second image data. The same may be true for the first image data. Further, some portions of the object may be partly in focus on the first frame of the image data and partly in focus on the second frame of the image data. Consequently, by combining or comparing both frames with each other an overall image or overall data may be generated that includes more information (e.g. more details, depth information etc.) of the object as compared to a single image of the object or a stereoscopic image of the object.

Preferably, an image cycle is provided in which first image data and second image data are acquired subsequently. That is, the image cycle may include the first sequence and the second sequence. In one embodiment, a plurality of image cycles are performed subsequently. The frames taken during the image cycle for the first image data and the second image data may vary. For example, when there is movement in scene to be examined, the image controller may cause the image sensor to generate several (e.g., eleven) frames for the first image data and only one frame for the second image data. This allows to have the video stream smooth. In other words, the user may examine the scene without being interrupted or disturbed by the process of acquisition of second image data. Further, in a next image cycle, the acquisition process may be repeated with the first image setting being maintained constant (i.e., without change) but change the second image setting for the second image data. That is, in the two image cycles the first image data include frames having the first image setting constant and the second image data including frames each having different second image settings. For example, in one second, 55 frames of the first image data and 5 frames of the second image data may be obtained, wherein, all frames of the first image data may have the same first image setting and all frames of the second image data may have varying second image settings. For example, the image cycle may be configured like in the following:

    i. 1 st setting - 11 frames (first image data),
    ii. 2nd setting 1 frame (second image data),
    iii. 1st image setting 11 frames (first image data),
    iv. 3rd setting 1 frame (second image data),
    v. 1st setting 11 frames (first image data),

    vi. 4th setting 1 frame (second image data),

and so on. More precisely, the second image settings may be different for each frame. On the other hand, the first image setting may be kept unchanged for all frames.

One cycle may be considered as one cycle of image setting change from first image setting to second image setting and then from second to first image setting. It may comprise one set of first image data and one set of second image data. Preferably, the image setting change is fast enough such that only one frame is acquired in the first and/or second image each data. In other words, time image controller may be configured to cause the image sensor to acquire more than one frame in the second image data, only when the image setting change is not fast enough to do this within one frame.

According to an aspect of the present invention, the depth map generation may be done in real time while object under examination is visualized to the user. Moreover, the user is not disturbed by the generation of the depth information as the second image data used for the depth generation is acquired in the background. In this way, the imaging device may be minimalized and the instrument in which it is used can be compact. Further, the user may be provided with both, with a visualization of the object and with detailed depth information of the object. In addition, the depth information may be used for an automated or at least partly automated handling device such as robots. Accordingly, the automated handling device may be accurately controlled.

Preferably, the first image data comprises at least 20 images per second. In other words, at least 20 images per second are taken to capture first image data during active intervention, for example.

Preferably, the first image setting and the second image setting differ at least in the focal distance and/or aperture. That is, the first image data and the second image data each have a different focal distance (i.e. a different focus). Preferably, the focal distance of the second image data may be suitable to create a depth map (e.g. to only partially focus the object). On the other hand, the focal length of the first image data may be suitable for bringing the entire object into focus. The first image data may have a focal distance greater than that of the second image data. Accordingly, the first image data and the second image data may be each suitable for their specific purpose.

Preferably, the data processor is configured to output a command to the image controller indicating which second image setting to set and/or when to control the image sensor to acquire second image data. That is, the data processor may demand to acquire the second image data. In other words, the user does not set or demand the second image data. The data processor may control the generation of the second image data. Accordingly, the data processor may control the generation of the second image data in accordance with a predetermined pattern. For example, the second image setting may be adapted to sample or scan a spatial region for an object or part of an object that is in focus in the second image data. Once such sharp portion is detected by the data processor, in a next acquisition of the second image data, the second image settings may be set to scan the surrounding of the sharp portion. This way, the generation of the second image data used for generating the depth map may have an increased efficiency. Further, the process of generating second image data may be automated. Further, the data processor may be configured to output a command to a timing controller. The timing controller may be configured to set a timing for acquisition of second image data.

Preferably, the command is based on the depth map generated by the 3D engine. That is, the result of generating the depth map can be evaluated to determine what further information is needed. For example, the depth map may have a gap or an area where there is little depth information. In this case, the data processor may adjust the second image data to obtain further depth information in that particular area. For example, the data processor may adjust the focal distance so that the second image data is sharp in that area. Accordingly, the accuracy of the depth map can be improved.

Preferably, the data processor is configured to separate the first image data and the second image data into two independent image data. The data processor may extract the second image data from the image stream of the first image data and the second image data. Further, the data processor may get the image setting corresponding to the respective image data. According, the image data may be supplied as an image data stream and may not be differently transferred from the sensor. Hence, an acquisition of both image data may be switched quickly.

Preferably, the imaging device further comprising the imaging sensor, preferably one single imaging sensor. The one single sensor may provide the advantage that the whole system may be very compact. In addition, there are no additional optical paths and mechanics necessary.

Preferably, the imaging device further comprising the 3D engine. The data processor may pass the image data to the 3D engine. The 3D engine may extract the depth information from the image data to do the 3D reconstruction. Further, the 3D engine may provide the depth map to a navigation engine that can corelate the data with 3D image derived from CT or MRI or other means. In addition, the 3D engine may also provide the depth map to a surgical robot controller. The depth map may also be provided to stereo image engine which may convert 2D image to 3D stereo image. This data may be provided to the image viewer and may thus be shown to the user either in 3D or 2D or as hologram.

Preferably, the 3D Engine is configured to determine an error of the depth map. The 3D engine may be configured to estimate an error of the depth map based on the sharpness of the image data received and of images of known objects such as estimates, etc. That is, the image data may include information of objects that have a known dimension, spatial position and/or size, for example. Accordingly, the 3D engine may compare the known information with the determined depth map. The difference between the known information and the determined depth map may be the error of the depth map. For example, the second image information may include information of a tool being in the field of view. The 3D engine may know the dimension of the tool so as to determine the real extension of the tool in the third direction (i.e., the z-direction). Such known dimension may then be compared to the determined dimension of the depth map. The known information may be any information that enables a comparison of the determined depth map with the reality. Further, the 3D engine may forward the error and the respective areas to the data processor. The data processor may then set the timing and second image setting for next acquisition of the second image data. That is, the 3D engine and the data processor may communicate in bidirectional way. This way, a feedback control may be implemented to increase the accuracy of the depth map. Accordingly, the depth map may be checked and further improved in accuracy in real time. In addition, the 3D engine may forward the error to the data processor.

Preferably, the image controller is further configured to control the second image settings based on the error. That is, the 3D engine may forward the error and the respective areas to the data processor. The areas may be that portion of the field of view included in the second image data that has the error (i.e., the difference between the real dimension and the determined depth map). The data processor may then set the timing and second image setting for next acquisition of the second image data. That is, the 3D engine and the data processor may communicate in a bidirectional way. This way, a feedback control may be implemented to increase the accuracy of the depth map.

Preferably, the image controller is further configured to control a light source which is configured to illuminate the object. The light source (illumination system) may be adjustable so as to illuminate an area which may be imaged by the image sensor (i.e., the field of view). Accordingly, the image data may be appropriately generated even in bad light conditions. The illumination system may be preferably a LED light source. In addition or alternatively, the illumination system may comprise a laser based light source. Further, the light source may be an external light source which may be controlled by the image controller. The illumination may also be achieved by lamps integrated within the optical system such as light source on a tip of an endoscope, for example. More specifically, the image controller can be configured to adjust the illumination, wavelength and/or illumination duration. In this way, the acquisition of the image data can be realized according to the desired image properties (i.e., the first image data may have different requirements with respect to lighting as compared to the second image data). Preferably, the image controller may be configured to adjust the light source such that it is adapted to the current image setting. This means that if the aperture is increased, less light may be required to achieve an optimal exposure. Accordingly, the image setting may be made in an appropriate manner, even if the boundary conditions change. The aperture may be fully opened in case of low lighting conditions to generate a better image.

Preferably, the imaging device comprises the light source configured to illuminate the object. Accordingly, an integrated device may be realized. As a result, the handling may be further facilitated as only one device has to be handled.

Preferably, the light source is configured to emit light having different wavelengths, preferably green wave length. The light emitted may be a multiplexed light which outputs different wavelengths. The kind of wavelength may be controlled by the image controller. The wavelength may be adjusted on demand or in planed sequence. For example, the wavelength may be varied for calibration purposes of the device. For example, the calibration should consider the performance of the entire system at different focal distances and over the entire sensing area over a range of wavelengths. The wavelength of the light may be changed for acquisition the second image data. In this way it is possible to obtain better contrast of structures. For example, green wavelengths are able to bring out better contrast of blood vessels.

Preferably, the image controller is further configured to control a lens and/or an aperture, preferably based on the image settings. That is, the lens and/or the aperture may define the image settings. That is, the lens may be responsible for the focal distance (i.e., the focus) or the magnification (zoom). The aperture may be responsible for the depth of focus (i.e., the area in the depth direction which is sharply depicted in the image data). Accordingly, the lens and/or aperture may be controlled in accordance to the first image setting and the second image setting. In the first image setting, it may be advantageously to set the aperture such that the depth of focus is large (i.e., the aperture is relatively closed). On the other hand, it may be advantageous to set the aperture in the second image setting so that the depth of focus is relatively small, so that both a sharp part of the object and an out-of-focus part of the object are included in the second image data. Preferably, the Imaging device comprises the lens and/or the aperture. A larger aperture will allow more light, therefore faster shutter speed and reduced motion blur.

Preferably, the lens and/or the aperture is a liquid lens. Implementing the liquid lens the focal distance may be changed. As liquid lenses can change their focus in short time, preferably in 10 to 20 ms. The liquid lens is compact in dimension. The imaging device may include an optical zooming lens. The optical zooming lens may be implemented as liquid lens as well. The zooming function may be activated on demand by the user or automatically determined by the data processor. Therefore, optimum magnification may be obtained without going to close to operating area. There are various principles for the realization of electrically controllable liquid lenses. For example, two-phase liquid lenses and/or single-phase liquid lenses may be implemented. One concept works with two immiscible liquids (aqueous and oil, respectively) of as similar a density as possible, but with different refractive indices and electrical properties in a chamber. An electrical voltage of up to 60 volts (between a ring electrode in the chamber and a transparent indium tin oxide (ITO) electrode on the outside of the chamber window) controls the electrical field, which changes the contact angle of a drop of the polar liquid (water) on the inside of this transparent chamber window via the effect of electrowetting. As a result, the hemispherical droplet becomes wider on the inner wall and thus reduces the curvature of its surface (the refractive phase interface), causing the lens to change its focal length. This allows focusing or zooming to be realized. Four radially arranged electrodes also enable a targeted wedge-like distortion or shifting of the drop. This makes it possible to achieve optical image stabilization in the case of a shaky camera or moving object with the help of the (auto) focus liquid lens. This technique was introduced by Varioptic in February 2010. Another concept uses MEMS (micro-electro-mechanical system) to deform a transparent membrane and thereby give a volume of liquid a different shape that has the desired refractive effect. Liquid lenses may be made particularly small as lenses with variable refractive power, which is especially necessary for cameras in flat mobile phones. They are fast, energy-saving and vibration-resistant. The solution of salts (such as potassium sulphate) in the water of the lens makes it conductive and lowers its freezing point as well as its vapor pressure sufficiently to ensure wide temperature ranges for operation (-20 to +60 °C) and storage (-40 to +85 °C) (i.e. the water neither freezes nor evaporates). The following technical data are typical: 8 mm diameter, 2 mm thickness, 2.5 mm optical aperture with a refractive power adjustable between -2 ... +10 dpt adjustable refractive power, Response time around 1 ms.

Further, the liquid lens may be an electrowetting liquid lens having an integrated adaptive liquid iris (ELL-LI) with adjustable radial aperture and axial position. Firstly, the liquid iris and the liquid lens may be integrated in the same chamber together, and the liquid iris and the liquid lens may share the same conductive liquid part. By changing the amount of liquid, the contact and fusion of two liquid-liquid interfaces in a three-layer liquid system may be changed to naturally form a liquid iris ring, which is attached to the side wall of the cylindrical chamber. The contraction and relaxation of the upper and lower boundaries may be achieved by changing the applied voltage and thus adjusting the aperture of the iris ring. Moreover, the electrodes of the sidewall dielectric layer, where the liquid iris is located may be segmented such that the iris can be subjected to upward or downward forces in the vertical direction, which means the iris can move up and down in the axial direction. Therefore, it can be used as an axially adjustable diaphragm in the optical system to realize the change of field of view and possible aberration modulation. Experiments show that the lens with adjustable radial aperture and axial position has the functions of depth of field expansion and field adjustment, and has a great application prospect in imaging systems, such as camera imaging systems. This technology is described in Optics & Laser Technology Volume 169, February 2024, 110023 "Electrowetting liquid lens integrating adaptive liquid iris" by Jin-Bo Xu, You-Ran Zhao, Rong-Ying Yuan, Xiao-Wei Li, Chao Liu and Qiong-Hua Wang. The article is available online since September 6, 2023 and is incorporated herein by reference.

Preferably, the image controller is further configured to control a piezo drive configured to change a position of the image sensor and/or the lens, based on the image settings. By changing of the position of the image sensor and/or of the lens, the image setting may be adjusted or changed. Accordingly, the image setting may be adjusted very quickly. The position change may be realized by changing the distance between sensor and part of the optics using piezo crystal. A fast mechanical movement can also be used to achieve change of focal distance in short time. This can also be achieved by using fast acting mirrors that project the image on two different sensors. The fasting acting mirror or mirror assembly may also modify the optical path length on one imager sensor to have the same effect. The piezo drive may be realized as a piezo crystal on which either the imaging sensor or focusing element is mounted. Further, the imaging sensor and the focusing element may be mounted on a piezo drive, respectively. The piezo drive may be more accurate but it is bulkier than a liquid lens. However, the piezo drive is more accurate and therefore has better repeatability. The liquid lens is more compact and can have faster response time. The piezo drive uses the piezoelectric effect which may be explained by a change in geometry. In general, all ferroelectric materials with a permanent electric dipole are also piezoelectric, for example barium titanate and lead zirconate titanate (PZT). However, only some of the piezoelectric behave ferroelectrically. Piezoelectric crystals may be used as piezo electric drive. For example, as a piezoelectric crystal the $\alpha$-quartz trigonal crystal structure formed by quartz is used, which is stable up to 573 °C. Further, lithium niobate may be used and has higher piezoelectric constants than quartz and is used for the piezoelectric drive Gallium orthophosphate may be also used. This material is similar to quartz, but has higher piezoelectric constants and better temperature stability. It is stable up to over 900 °C. Other piezoelectric crystals are berlinite, minerals of the tourmaline group, seignette salt and all ferroelectrics such as barium titanate (BTO) or lead zirconate titanate (PZT). Preferably, the imaging device comprises a piezo drive configured to change the position of the image sensor.

Preferably, the Imaging device comprises the lens and/or the aperture, wherein the lens and/or the aperture is preferably a piezo drive.

Preferably, the image settings comprise aperture, depth of focus, light, wavelength, shutter speed and/or focal distance. Further, the image settings may comprise a brightness. Accordingly, the image setting may influence the information of the image data. This way, the first image data and the second image data may differ from each other. The aperture may be changed to alter the depth of field (i.e. the area in the depth direction where the image data is in focus). The depth of field may be changed to alter the extent in the depth direction in which the image is in focus. The light (e.g., the light intensity and/or the wavelength) may be changed to emphasize some features of the object and/or to provide a sufficient lighting to the object to be imaged. The shutter speed may be changed to adapt the image setting to changed other parameters. For example, if the aperture is increased, the shutter speed may be reduced to ensure sufficient exposure of the image data. The focal distance (i.e. the area where the image data is in focus) may be adjusted to scan the object in the field of view. Accordingly, the first image data (e.g. for visualizing the object) and the second image data (e.g. for creating the depth map) may be acquired in a way that is optimal for their respective purposes.

Preferably, the imaging sensor comprises a shutter having an adjustable shutter speed. Accordingly, the imaging sensor may be adapted to varying image setting. Thus, sufficient acquisition of image data may be secured.

Preferably, the first image setting is to be inputted by the surgeon via a user interface. In other words, the first image setting may be determined by the user or user need. It may also be predetermined based on user opinion or preferences based on intended use. In other words, the first image setting may not be determined automatically without consideration to user need. In this way, the user may directly influence the visualization of the first image data. This means that the user may orientate himself on the visualized first image data when examining the object. In this way, the visualization may be adapted to his personal preferences and/or to the current boundary conditions, such as the light in the operating theatre.

Preferably, the second image setting is set automatically. This means that the second image setting may be set without direct influence from the user. On the other hand, the second image setting may be solely set by the data processor. This way, the second image setting may be determined based on requirements that are necessary to determine the depth map. In addition, the second image setting may be updated so that they are always ideal for creating the depth map in the given conditions, even in case boundary condition change.

Preferably, the imaging device comprises a navigation engine configured to generate overlay information based on the depth map. That is, the depth map may be combined with other images such as 2D-images. Accordingly, further information may be generated based on the depth map.

Preferably, the navigation engine is configured to corelate the depth map with 3D image data derived from CT or MRI. In other words, the 3D engine may provide the depth map to the navigation engine that may corelate the data with 3D image derived from CT or MRI or other means. In this way, automatic navigation of a tool using the imaging device may be implemented. For example, in case the imaging device is used together with an endoscope, the navigation engine may determine the spatial position of the endoscope based on the 3D images and the depth map. In other words, the 3D construction (i.e., the depth map) may be used for various purposes. Such as converting a video image to a stereo image for visualizing by user. It may be also used for combining the 3D reconstruction with 3D reconstruction derived from CT scan or MRI. The information may be used to provide context specific information to the user by way of augmented reality or video or audio feed back. The feedback may be provided by any other means. It may be also used to stop accidents or injuries.

Preferably, the imaging device comprises a stereo image engine configured to generate a 3D model based on the depth map and 2D images in particular derived from the first image data. That is, the image data will also be provided to the stereo image engine which is configured to convert the 2D image data (i.e., the first image data) to 3D image such as stereo image. This data may be provided to the image viewer. Accordingly, the user may be provided with a 3D visualization of the object under examination.

Preferably, the stereo image engine 3D model is configured to generate an Augmented reality, a video and/or audio feedback based on the depth map. Accordingly, a feedback control may be implemented. The augmented reality, the video and/or the audio feedback may support the user in examining the object.

Preferably, the imaging device comprises a surgical robot controller configured to control a robot controlling a tool, in particular an endoscope, based on the depth map. The robot may be an automated device for carrying out a surgical process. The tool may be used to analyze and/or manipulate the object. The robot may be also referred to as a robotized system. The imaging device may or may not be part of such robotized system. In the robotized system, the imaging device may be held steady. If, on the other hand, a user holds the imaging device in his hand, it is not always guaranteed that the imaging device is held still. In the latter case, the image data may be captured with minimum motion blur. When the imaging device is held by the robotic system, it can be ensured that the image data capture has minimum motion blur and is therefore very sharp and has more distinguishing features that can be used for 3D reconstruction. The instruments can be moved within the field of view in such a way that they are captured by the imaging data. However, if it is ensured that the imaging device is held still or if the movement of the imaging device is known, the information from the instruments can be used to create the 3D reconstruction. More specifically, the dimensions of the instruments are generally known and this data can be used to determine the depth map even in the case of movement. In addition, such information may be used to calibrate the imaging device or the check the determined depth map. The imaging device may also be held steady by means other than robot, for example using a mechanical holder.

Preferably, the frame rate of the image data is at least 20 FPS, preferably between 20 and 100 FPS. FPS may mean frames per second which may be also referred to as frame frequency or frame rate. This term may refer to the number of frames that are recorded or played back per period of time and is usually expressed in the unit fps (frames per second). The frame rate of video is usually between 20 to 100 frames per second. A frame rate of less than 30 per second can be used for certain purposes, e.g. bleeding, fluorescence imaging, when no active tissue is manipulated, etc.. Preferably, the imaging device is configured to control the image sensor so as to work at 60 frames per second. In this case, it was found that the second image data could be captured without disturbing the user in their examination work.

Preferably, the first image data is captured during a first sequence and the second image data is captured during a second sequence. Each sequence may describe a time span in which a specific control of the imaging device is executed. For example, the second sequence may include changing focus (i.e., changing to second image setting), capturing second image data, switching focus back to main focus (i.e., to the first image setting). As this second sequence runs in a very short time, the user can work without his workflow being affected. All image data which do not have the main focus (i.e., the first image setting) may be considered as second image data. In addition, second image data with different focal distance can be recorded in the second sequence. The images acquired with main focus (i.e., with first image setting) may be considered as first image data. The first sequence and the second sequence may alternate continuously. For example, 30 frames per second may be captured in the first sequence (i.e., first image data) and 30 frames in the second sequence (second image data). If there is not enough light, for example, 30 images per second can be captured in the first sequence and 5 images in the second sequence. In short, any combination may be implemented. Thus the imaging device may be adapted to the current boundary conditions.

Preferably, the frequency of the second sequence and/or the variation of the second image setting, in particular the focal length, may be determined dynamically depending on the degree of uncertainty. The uncertainty may be determined by the data processor. More specifically, the uncertainty may be determined based on the depth map. That is, the more errors or gaps there are in the depth map, the greater the uncertainty. In this case, acquiring more second image data may improve the accuracy of the second image data and thus the depth map. In addition, the first image setting may be also changed based on the degree of uncertainty. In more detail, a specific allowance or subtraction to the main focal distance (of the first image setting) may be implemented to acquire the first image data. The change of the image setting may also be adjusted according to a certain pattern. In such a pattern, an increment in which the image settings are changed may be specified. After the image data has been acquired, it may be determined how great the uncertainty is. Then image settings can be set where the uncertainty is minimal. Indications derived from other data such as instruments can be used to determine uncertainty. Accordingly, an improved accuracy of the image data may be achieved.

Preferably, more frames are taken during the first sequence than during the second sequence. This is particularly advantageous when the user manipulates the object. In this way it may be ensured that the user is not disturbed in his examination work based on the first image data. In other words, the dynamic acquisition of the first image data may be sufficient to provide the user with initial image data that gives a good impression of the object under examination. At the same time, the second image data is acquired to create the depth map.

Preferably, the image controller is configured to alternate between the first sequence and the second sequence. In other words, the first sequence may be performed as long as enough first image data has been acquired to provide the image viewer with enough data to visualize the object. Then the image controller may switch back to the second sequence to acquire second image data. The frequency of alternation may be predetermined. Alternatively, the alternation frequency may depend on the user's examination activity. For example, if the user is moving a tool or the area of interest is in motion, it may be necessary to provide more initial image data to achieve a fluid visualization of the object. For example, the image controller is configured to control the image sensor so as to capture 30 frames of the first image data and subsequently less than 30 frames of the second image data. This frequency was found to give the best results in terms of depth map accuracy and ease of use. In normal use of the imaging device, the first sequence may be no less than 20 per second. The second sequence may generally comprise fewer frames than the first sequence. It is also possible that no second image data is acquired if the 3D reconstruction of the scene is already considered good enough by the data processing equipment or the user.

Preferably, the image controller is configured to recognized whether the endoscope is moved and to determine movement information, wherein the image controller is configured to change the image settings based on the movement information. If the imaging device detects that the user is not actively using the device or tool, the image controller may cause the image sensor to continuously acquire second image data with different focal distances. This is the case, for example, when the user changes instruments or waits for another event in surgery. In this way, the 3D reconstruction may be better optimized in a short time without disrupting the workflow. The system may increase the amount of second image data (e.g., the number of frames) at the beginning of use to develop a good initial 3D reconstruction and have a lower number during later use. In this way, a fast generation of the depth map is ensured.

Preferably, the image controller is configured to change the second image settings in accordance with a predetermined pattern. For example, depending on the type of examination, a predetermined pattern may be used. For example, a pattern established in a previous examination may be used again. The pattern may be stored in a database and used based on the specific examination to be performed. In this way, the use of the imaging device may be made more efficient. The pattern may be indicative of a change of focus (i.e., a focus shift). Accordingly, the object may be scanned, in particular with respect to its depth direction.

Preferably, the image sensor is configured to capture image data having different wavelength. Accordingly, the image setting may be changed by changing the wavelength received by the imaging sensor. For example, when imaging longer wavelengths such as near infrared (NIR), the focal distances change due to difference in coefficient of refraction of visible light and NIR light. In this way the focus may be shifted so as to acquire second image data. The focus shift technique may be used to generate second image data and the reference focal distance for the respective wavelength. This is particularly useful for 3D reconstruction of second image data with longer wavelengths.

Preferably, the imaging device comprises at least one mirror configured to redirect the light to be received by the image sensor so as to change the image setting, based on a command of the image controller. In other words, a mechanical movement may be used to achieve change of image setting in short time. This may be achieved by using fast acting mirrors that are configured to project the image on two different sensors. The fasting acting mirror or mirror assembly may modify the optical path length on one imager sensor. In this way, the image setting may be changed from first image setting to second image setting without disturbing the user.

Preferably, the second image data is not directly shown to the user. That is, the second image data may be solely used to generate the depth map. On the other hand, only the first image data may be visualized so as to be shown to the user. In this way, the respective image data may be set exactly so that they are optimal for the respective application (i.e. first image data for visualization and second image data for generating the depth map). This is made possible by the image controller, which may change the image setting so quickly that the visualization of the first image data is not disturbed by the small gap in which the second image data is captured.

Preferably, the image controller is configured to improve the sharpness of the first image data based on the second image data. That is, the second image data collected at different focus may be used to improve sharpness of the first image data. For example, a spatial position of the object may be judged based on the second image data. Then, the image setting of the first image data may be adjusted to take into account the spatial position of the object. In this way, the visualization of the first image data may be improved and made more efficient because the information obtained by analyzing the second image data may be applied to the first image data (e.g. by adjusting the first image setting). For example, when the location of a tool tip (e.g., of an endoscope) is known, the first image settings may be configured to show the contact point of the tool tip to tissue with high sharpness. Alternatively, the intended tissue can be better focused because of known depths.

According to a further aspect of the present invention, an endoscope is provided having an imaging device according to any one of the above embodiments. In other words, the endoscope may include a camera head comprising the above imaging system. The camera head may accommodate the imaging device and may be connected to the endoscope via a camera adapter. A focusing ring may be arranged on the camera adapter of the endoscope so as bridge the camera head and a telescope of the endoscope. The telescope may be a shaft of the endoscope. An optical channel may be arranged within the shaft. Further, the shaft may be flexible so as to allow the optical channel to be rotated at a distal end of the endoscope (e.g. the telescope) or may protrude from the distal end of the endoscope. Further, the endoscope may comprise a light source (lamp). The light source may be adjustable so as to illuminate an area to which the distal end of the optical channel is directed. Accordingly, the first and second image data may be appropriately generated even in bad light conditions. The light source may be preferably a LED light source or laser light source. As a result, an endoscope may be provided that may acquire at least two focus shifted image data using a single image sensor. As a result, the endoscope may have a highly integrated design and may be used in a wide field of applications.

According to an aspect of the present invention a system is provided comprising an Endoscope according to any of the above embodiments, and a tool configured to be used within the field of view of the imaging sensor, wherein the tool has a mark wherein the image controller is configured to change the image settings based on the mark within the field of view. The tool or Instrument may be usually moving. Therefore, the tool may or may not be in focus. In addition, the tool may comprise a mark, for example a barcode, to recognize it. For example, when the tool is not in focus, the marking may not be visible. The image controller may help to identify the marking even when it is out of focus in in the first image data. However, the marking may be in focus in the second image data (i.e., in any frame of the second image data). In addition, the marking may be of known shape, size which will also help to identify the depth. Further, the marking may be used to calibrate the determination of the depth map.

According to an aspect of the present invention a method for imaging an object is provided comprising: acquiring first image data of the object, having first image setting, and second image data of the object, having second image setting, wherein the first image setting and the second image setting differ from each other, switching the image settings between the first image setting and the second image setting in less than 100ms, forwarding the image data to a 3D engine, and generating a depth map of the object based on the image data. Features and advantages disclosed in connection with the device may also be applied to the method and vice versa. The image data may include not only the image data, but also associated image data settings.

Image data acquired using image settings that are primarily decided based on a user's need is referred to as first image data. Image data acquired not having image settings primarily decided based on a user's need is referred to as second image data. Image settings for the first image data may remain generally stable when the scene (i.e., the object) under imaging is stable. The second image settings may not remain stable even when the scene is stable. The second image data may be less than first image data.

The user is mainly shown images based on the first image data. The user may be shown a small number of images based on the second image data if it is deemed to be not disturbing. This may not be more than 30% of images shown during an intervention. These images may be shown in 2D directly from data processor or they may be shown in 3D / 3D as output from 3D engine. They may also be shown in 2D as output from navigation engine.

A duration of the acquisition of the second image data is shorter than 300 ms when there is activity in the image (i.e., active intervention) to reduce inconvenience to the user.. The activity may be the movement of the image sensor, the instrument, the manipulation of tissue or any other intentional activity that requires the user to focus on the image to perform the tasks.

If the image settings are different, but the difference is of a kind that does not affect the user, they are considered first image setting or first image data. In the case of a transition phase from one setting to another, some images may be part of the first image data (first sequence) and some part of the second image data (second sequence), depending on the speed of the transition and its influence on the user.

In addition, the present invention is also directed to a use of the imaging device according to any one of the above embodiments in a surgery tool such as an endoscope.

According to an embodiment of the present invention an imaging device is provided. The imaging device may be also referred to as a fast focus mechanism. The imaging device may be integrated in an optical system or a holding system. The image controller is incorporated in the system. This controller may be hardware or software or both. The controller may send commands to imager (e.g., an image sensor) about how and when to capture the image data with shifted focus (second image data) than the main focus (first image data). This image includes timing as well as focal distance or focal shift. The main focus is decided by surgeon either manually or automatically. It is the region of his main working, manipulation or dissection. In case of open surgery, it will be mainly the central part of the image. The surgeon will mainly see the images with main focus (first image data). This may be fixed distance or varied during the surgery. The controller may trigger focus change action or know when such change will happen. The controller may pass on the information to the data processor. The data processor may extract the images from the image stream, may know the image capture parameters. The parameters may be focusing distance or the difference, light settings, timing, etc. The data processor will pass this info to the 3D engine. The 3D engine may extract the depth information from this do the 3D reconstruction. The sensor, the controller, the data processor and 3D engine may be connected to each other and be able to communicate each other either bidirectionally or unidirectionally. The 3D engine may estimate errors based of sharpness of images received, from the images of known objects such as estimates, feedback from a connected robotic system, etc. And it may inform the error and the respective areas to data processor. The data processor may decide the timing and focal distance or focal shift for next image capture based on the error as well as other parameters described below. The 3D engine may provide the depth map to the navigation engine that may corelate the data with 3D image derived from CT or MRI or other means. It may also provide the data to surgical robot controller. The data may also be provided to a stereo image engine which may convert 2D image to 3D image. This data may be provided to the image viewer.

According to an embodiment of the present invention, the frame rate of video is usually between 20 to 100 frames per second. Frame rate of less than 30 per second is usually used for specific purposes such as bleeding situation, fluorescence imaging, when no active tissue manipulation is happening, etc. When the imager is typically working at 60 frames per second. By using the fast focus mechanism, the focus may be changed within less than 100 milliseconds, one or more images may be captured and focus changed back to main focus. All this may happen within less than 300 milliseconds at most. This sequence of changing focus, capturing image, switching focus back to main focus is called as second sequence. As all this second sequence happens in very short time, the user may work without affecting his workflow. All images which do not have the main focus may be called second image data. In one second sequence there may be one image or several images with different focal distances. The images are taken with main focus are called first image data (i.e., during the first sequence).

Liquid lens may change their focal distance at a very fast speed. This property of liquid lens is used in this embodiment. Also, piezo drives may act very fast over short distances and maintain high accuracy. In typical situation, a refocus mechanism may achieve targeted focus in 20ms, the image may be taken in 20ms and focus can be shifted back to main focus in 20ms. The total process may happen within 60 ms. As this period is very short, it may not be noticed by the user even if there is active manipulation of the tissue.

The 3D construction may be used for various purposes. For example, the video image may be converted into a stereo image to make it visible to the user. It may also be used for combining the 3D reconstruction with 3D reconstruction derived from CT scan or MRI. The information may be used to provide context specific information to user by way of augmented reality or video or audio feed back. The feedback may be provided by any other means. It may also be used to stop accidents or injuries. In robotized systems, the imager (e.g., the sensor) may be held steady. Therefore, the images may be captured with minimum motion blur. This enables capture of very sharp images with more distinguishing features that can be applied for 3D reconstruction. Instruments may be moving in the image but their dimensions are generally known and this data can be used even in case of movement.

According to a further embodiment, the first sequence and the second sequence may be continuously alternating when it is practically feasible. For example, there may be 30 images per second in first sequence and 30 images of second sequence. When there is not enough light or the hardware or software is not fast enough, there may be 30 images of first sequence per second and 5 images of second sequence. In short, any combination can be used. When the system is in normal use, the first image data may not have a framerate of less than 20 per second. Second image data may have generally a framerate lower than that of first image data. It may also be possible that no second image data is taken if the 3D reconstruction is already considered by the system or the user to be good enough. When the device detects that the user is not actively using the system, the device may take several images with different focal distances continuously as second image data. An example of this is when user is changing the instruments or waiting for some other event to happen in surgery. This way, the 3D reconstruction may be better optimized in short time without disturbing the workflow. The system may increase number image B in the beginning of use to develop a good initial 3D reconstruction and have smaller number during subsequent use. The frequency of second sequence and/ or the variation in focal distance may be dynamically determined depending on degree of uncertainty. This can also be a specific addition or deduction to the main focal distance which is used for capturing main image (first image data). This can also be a planned fixed pattern. Cues derived from other data such as instruments can be used to determine uncertainty. When imaging longer wavelengths such as near infrared (NIR), the focal distances change due to difference in coefficient of refraction of visible light and NIR light. The technique of shifting focus may be used to generate sharpness data and the reference focal distance for the particular wavelength. This be applied to do 3d reconstruction of longer wavelength image.

According to an embodiment, the device needs to be calibrated depending on the wavelengths to be used. Multiple calibrations may be needed at different wavelengths if such light is to be used or fluorescence is expected. The light used may be a multiplexed light which outputs different wavelengths on demand or in planed sequence. The calibration may consider the performance of entire system at different focal distances and over entire sensing area over range of wavelengths. To get more accurate 3D reconstruction, the aperture may by dynamically varied. For example, the first image data may be captured with smaller aperture and second image data may be captured with larger aperture. Larger aperture may allow smaller depth of focus and higher accuracy for depth estimation. Also, it may allow more light to have the image captured in shorter time. This minimizing impact on workflow or to capture of multiple images with varying focal distance. Most optimal way of changing the focal distance may be by use of liquid lens. As liquid lenses may change their focus in short time of 10 to 20 ms. This may also be done by changing the distance between sensor and part of optics using piezo crystal. A fast mechanical movement may also be used to achieve change of focal distance in short time. This may also be achieved by using fast acting mirrors that project the image on two different sensors. The fasting acting mirror or mirror assembly may also modify the optical path length on one imager sensor to have the same effect. When using piezo crystal, either the sensor or focusing element may be mounted on piezo. Piezo system may be more accurate but it is more bulky than liquid lens arrangement. However piezo is more accurate and therefore has better repeatability. Liquid lens design is more compact and can have faster response time.

According to a further embodiment, the light may be further optimized for capturing second image data. For example, the light intensity may be increased for short time to get better image in short time. This may help keep the heat in control without reducing performance. Further, the wavelength of light may be changed for second image data to obtain better contrast of structures. For example, green wavelengths are able to bring out better contrast of blood vessels. Sometimes there are no distinguishing features in the field of view. A random pattern may be projected onto the image for the duration of the recording of second image data. This may contribute to a better 3D reconstruction without disturbing the user. These functions may be activated automatically or on request by user. The second image data may not directly be shown to the user. The results of the analysis of the second image data may be shown to the user.

Instruments are usually moving. Therefore, they may or may not be in focus. There is increasing trend to mark the instruments, for example with barcode, to recognize them. When the instrument is not in focus, the marking may not be visible. The fast focus mechanism can help identify the marking even when it is out of focus in main focus. The markings may be of known shape, size which will also help to identify the depth. The second image data may be used to improve sharpness of first image data.

The device may incorporate an optical zooming lens. The lens may be made of liquid lens as well. The zooming function may be activated on demand by user or autonomously determined by the image controller. Therefore, optimum magnification can be obtained without going to close to operating area.

More than one image sensor may be used for imaging. In this case, any of the image sensors may be considered as main image sensor to make the first image data. Basically the image sensor which has settings as per user need will be the main sensor and image date from that sensor will be considered as first image data.

The advantages and features described in connection with the device are also applicable to the method and vice versa. Wherever not already described explicitly, individual embodiments or their individual aspects and features can be combined or exchanged with one another without limiting or widening the scope of the described invention, whenever such a combination or exchange is meaningful and in the sense of this invention. Advantages, which are described with respect to one aspect of the present invention are, wherever applicable also advantages of other aspects of the present invention.

Useful embodiments of the invention shall now be described with reference to the attached figures. Similar elements or features are designated with the same reference signs in the figures. In the figures is:

Fig. 1    is a schematic diagram of an imaging device according to an embodiment of the present invention.

Fig. 2    is a schematic visualization of an endoscope according to an embodiment of the present invention.

Fig. 3    is a schematic visualization of an endoscope according to another embodiment of the present invention.

Fig. 4    is a schematic diagram of an imaging device according to an embodiment of the present invention.

Fig. 5    is a schematic and perspective visualization of an endoscope according to an embodiment of the present invention.

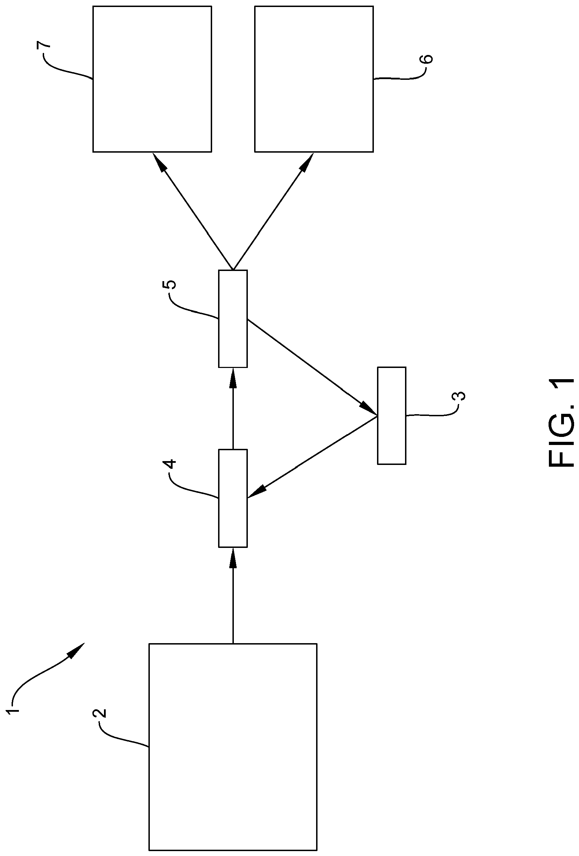

**Figure 1** is a schematic diagram of an imaging device 1 according to an embodiment of the present invention. The imaging device 1 comprises an image controller 3 and a data processor 5. The image controller 3 is a computer system configured to receive data, to process data and to output data. The output data of the image controller 3 may be commands for controlling other devices. The image controller 3 is configured to control and image a sensor 4. The image senor 4 is configured to acquire image data of an object 2 which is to be examined. The object 2 may be a part of the human body or of a machine which is to be assessed. The image sensor may receive light and may transfer the light information into image data.

The image sensor 4 is controllable by commands received from the image controller 3. The image sensor 4 is configured to acquire first image data of the object 2 having first image setting and second image data of the object 2 having second image setting. The first image setting and the second image setting differ from each other. In other words, the first image data and the second image data represent different images, at least due to the different image settings. The image sensor 4 is configured to transfer the image data to the data processor 5. The data processor 5 is configured to receive the first image data and the second image data and to forward first image data to an image viewer 7 and both first and second image data to a 3D engine 6. Further, the data processor 5 is configured to forward information to the image controller 3. In addition, the image controller 3 is configured to change the image setting between the first image setting and the second image setting in less than 100ms. Moreover, the 3D engine 6 is configured to generate a depth map of the object 2 based on the image data. The 3D engine 6 may be a computer-like apparatus and may use the image data as an input and the depth map as an output.

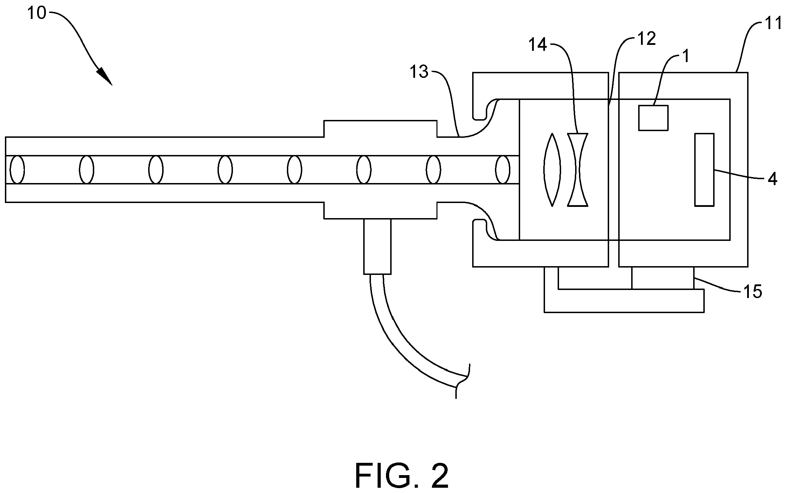

**Figure 2** is a schematic visualization of an endoscope 10 according to an embodiment of the present invention. The endoscope 10 comprises the imaging device 1 according to the above embodiment. Further, the endoscope 10 includes a camera head 11, a connecting member 12, and a shaft member 13. The imaging device 1 is positioned within the camera head 11. A focus mechanism 14 is positioned in the connecting member 12. The light information is transferred to the shaft member 13, the focus mechanism 14, towards the sensor 4 positioned within the camera head 11. The shaft member 13 may include a light terminal for connecting an external light source (not depicted in Fig. 2). In the present embodiment, the focus mechanism 14 includes a liquid lens. The liquid lens is configured to change the focal distance in less than 100ms. Accordingly, the first image setting and the second image setting may be implemented quickly without disturbing a user watching at the first image data. Optionally, a piezo drive 15 may be provided. The piezo drive 15 may be provided in combination with the liquid lens or may be provided as an alternative. The piezo drive 15 supports the camera head 11 so as to relatively move the sensor 4 with respect to the focus mechanism 14. In this way, the focal length may be adjusted. In more detail, by operating the piezo drive 15, the image controller 3 may change between the first image setting and the second image setting. The piezo drive 15 may be controlled in such a way, that the respective image setting can be implemented in less than 100ms. In addition, the piezo drive 15 is capable of setting the desired image setting very accurately. By using angled endoscope and rotating the shaft, it is possible to view larger area. When the image sensor 4 is independent of shaft, the shaft can be rotated independently of the image sensor. In this way, the image orientation is maintained for the user.

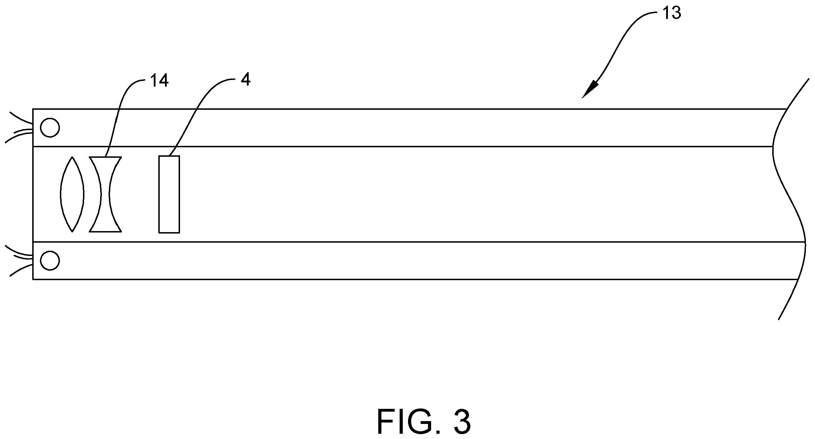

**Figure 3** is a schematic view for part of an endoscope 10 according to another embodiment of the present invention. The present embodiment differs from the previously defined embodiment in that the sensor 4 is provided within the shaft member 13. In addition, the focus mechanism 14 is also provided within the shaft member 13. The image sensor 4 is connected to the imaging device 1 via a wire or wireless. The focus mechanism 14 of the present embodiment includes a liquid lens, capable of adjusting the image setting in less than 100ms. When angled endoscope is used in this configuration, the image sensor 4 may be configured to rotate with the endoscope. The movement of the endoscope must be tracked. Either the sensor must be corrected mechanically or electronically or by software.

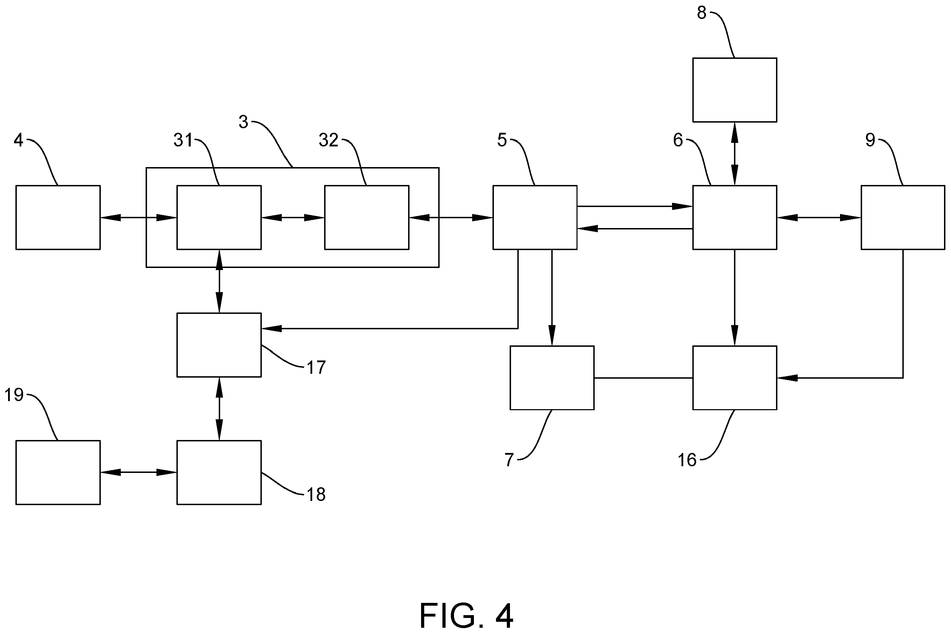

**Figure 4** is a block diagram depicting the general configuration of the imaging device 1 according to an embodiment of the present invention. On the left side of Figure 3, the image sensor 4 is depicted. The image sensor 4 is configured to convert light information into data information. The data information is forwarded to the image controller 3. The image controller 3 of the present embodiment includes a raw data processor and an image processor 32. The image controller 3 is configured to output processed images to the data processor 5. In addition, the image controller also outputs the image settings (i.e. first image setting and second image setting) to the data processor 5. The data processor 5 outputs the first image data and the second image data for further processing. In more detail, the data processor 5 outputs the first image data to the image viewer 7. The image viewer 7 is configured to realize the object for a user in 2D. The data processor 5 is further configured to output the first and second image data to the 3D engine 6. The 3D engine 6 generates a depth map based on the image data. The 3D engine 6 is further configured to output the depth map to a surgical robot controller 8. The surgical robot controller 8 is configured to control a robot which controls a tool or the endoscope 10. In addition, the 3D engine 6 is configured to output the depth map to a navigation engine 9. The navigation engine has configured to overlay the depth map with further image data received from external means, for example. Then, the overlay info is forwarded to a stereo image engine 16. The stereo image engine 16 may in addition receive information directly from the 3D engine 6. Then, the output of the stereo imagine engine 16 is forwarded to the image viewer 7 and could be depicted for the user in 2D or 3D. The 3D engine is further configured to determine an error of the depth map. The determined error may then be forwarded to the data processor 5. The data processor 5 may be in communication with the image controller 3 and provides a timing request based on the error determined by the 3D engine. In addition, a timing controller 17 may be provided which is configured to determine a timing when further second image data is to be acquired by the image sensor. In other words, the image controller 3 may control the image sensor 4 and the associated optical and mechanical system based on a feedback control. In addition, a light source controller 18 is provided. The timing controller 17 is configured to output the timing to the light source controller 18. The light source controller 18 is configured to control a light source 19. The light source controller and the timing controller 17 may be implemented in the image controller 3. The image processor, data processor, 3D engine, stereo image engine and navigation engine may be separate or combined. One computer processor or graphics processing unit may perform all of these tasks.

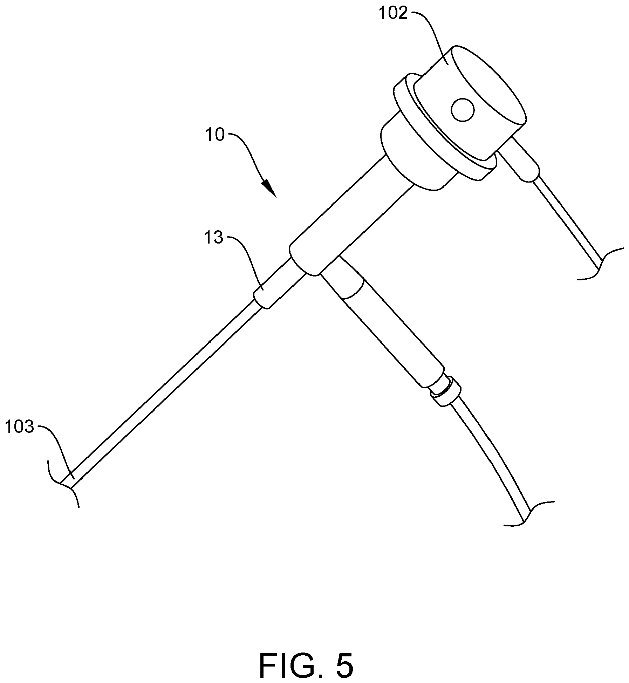

**Figure 5** is a schematic and perspective view of an endoscope 10 according to an embodiment of the present invention. The endoscope 10 includes the imaging device 1 within the housing 102. The endoscope has a shaft member 13, having a distal end 103. An optic channel is provided within the shaft member 13. The endoscope 10 includes a light source (not depicted in the figures) that is configured to emit light from the distal end 103 of the endoscope 10.

The above discussion is intended to be merely illustrative of the present invention and should not be construed as limiting the appended claims to any particular embodiment or group of embodiments. Thus, while the present system has been described in particular detail with reference to exemplary embodiments, it should also be appreciated that numerous modifications and other alternative embodiments may be devised by those having ordinary skill in the art without departing from the broader and intended spirit and scope of the present system as set forth in the claims that will follow. Accordingly, the specification and drawings are to be regarded in an illustrative manner and are not intended to limit the scope of the appendant claims.

Reference numerals:

| 1 | imaging device |
|---|---|
| 2 | object |
| 3 | image controller |
| 4 | image sensor |
| 5 | data processor |
| 6 | 3D engine |
| 7 | image viewer |
| 8 | surgical robot controller |
| 9 | navigation engine |
| 10 | endoscope |
| 11 | camera head |
| 12 | connecting member |
| 13 | shaft member |
| 14 | focus mechanism |
| 15 | piezo drive |
| 16 | stereo image engine |
| 17 | timing controller |
| 18 | light source controller |
| 19 | light source |
| 32 | image processor |
| 102 | housing |
| 103 | distal end |

**Claims**

1. Imaging device (1) for generating imaging data of an object (2), comprising:

   an image controller (3) configured to control an image sensor (4) to acquire first image data of the object (2), having a first image setting, and second image data of the object (2), having a second image setting, wherein the first image setting and the second image setting differ from each other,
   wherein the image controller (3) is further configured to switch the image settings between the first image setting and the second image setting in less than 100ms,
   a data processor (5) configured to receive the first image data and the second image data and to forward the image data to a 3D engine (6),
   wherein the 3D engine (6) is configured to generate a depth map of the object (2) based on the image data.

2. Imaging device (1) according to claim 1, wherein the first image setting and the second image setting differ at least in the focal distance and/or aperture.

3. Imaging device (1) according to claim 1 or 2, wherein the data processor (5) is configured to output a command to the image controller (3) indicating which second image settings to set and/or when to control the image sensor (4) to acquire second image data.

4. Imaging device (1) according to any of one of the preceding claims, further comprising an imaging sensor (4), preferably one single imaging sensor (4).

5. Imaging device (1) according to any of one of the preceding claims, further comprising the 3D engine (6), wherein the 3D engine (6) is configured to determine an error of the depth map.

6. Imaging device (1) according to any of one of the preceding claims, wherein the image controller (3) is further configured to control a light source (19) which is configured to illuminate the object (2)

7. Imaging device (1) according to any of one of the preceding claims, wherein the image controller (3) is further configured to control a lens and/or an aperture.

8. Imaging device (1) according to any of one of the preceding claims, wherein the Imaging device (1) comprises the lens and/or the aperture, wherein the lens and/or the aperture is preferably a liquid lens.

9. Imaging device (1) according to any of one of the preceding claims, wherein the image settings comprise aperture, depth of focus, light, wavelength, shutter speed and/or focal distance.

10. Imaging device (1) according to any of one of the preceding claims, further comprising a surgical robot controller (8) configured to control a robot controlling a tool, in particular an endoscope (10), based on the depth map.

11. Imaging device (1) according to any of one of the preceding claims, wherein the first image data is captured during a first sequence and the second image data is captured during a second sequence, wherein more frames are taken during the first sequence than during the second sequence.

12. Imaging device (1) according to any of one of the preceding claims, wherein the image controller (3) is configured to recognized whether the imaging device (1) is moved and to determine movement information, wherein the image controller (3) is configured to change the image settings based on the movement information.

13. Endoscope (10) having an imaging device (1) according to any one of the preceding claims.

14. System comprising:

an endoscope (10) according to claim 13, and a tool configured to be used within the field of view of the imaging sensor (4), wherein the tool has a mark wherein the image controller (3) is configured to change the image settings based on the mark within the field of view.

15. Method for imaging an object (2) comprising:

acquiring first image data of the object (2), having first image setting, and second image data of the object (2), having second image setting, wherein the first image setting and the second image setting differ from each other, switching the image settings between the first image setting and the second image setting in less than 100ms, forwarding the image data to a 3D engine (6), and generating a depth map of the object (2) based on the data.

FIG. 1

FIG. 2

EP 4 544 977 A1

FIG. 3

FIG. 4

FIG. 5

# EP 4 544 977 A1

**EUROPEAN SEARCH REPORT**

Europäisches Patentamt
European Patent Office
Office européen des brevets

**Application Number**

EP 23 20 5911

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2022/187509 A1 (TAVENIKU MIKAEL BROR [US] ET AL) 16 June 2022 (2022-06-16) * paragraphs [0063], [0070], [0076], [0079], [0080], [0083], [0093], [0097], [0109] * * figures 4, 5, 6A, 8 * | 1-4,7-9, 12,15 | INV. A61B1/00 A61B1/045 G06T7/571 H04N13/271 H04N23/50 |
| X | US 2015/271467 A1 (WEINSTOCK NEAL [US]) 24 September 2015 (2015-09-24) * paragraphs [0034] – [0036], [0043], [0048] – [0051] * * figures 3, 8, 14 * | 1-5,7-9, 15 | ADD. A61B1/04 A61B34/30 G06T7/00 |
| X | US 2022/293268 A1 (NISHIDE AKIHIKO [JP]) 15 September 2022 (2022-09-15) * paragraphs [0033], [0041] * * paragraphs [0048] – [0052] * * paragraphs [0066], [0092], [0109] * * paragraphs [0117] – [0122] * * figures 2, 3, 10, 11 * | 1-11, 13-15 | |
| X | US 2019/090753 A1 (CARSON JOSEPH [US] ET AL) 28 March 2019 (2019-03-28) * paragraphs [0019], [0020], [0027] * * figures 1, 2, 7 * | 1-4, 7-10, 13-15 | TECHNICAL FIELDS SEARCHED (IPC) A61B G06T H04N |
| X | US 2018/344145 A1 (KOZUB DANYLO [UA] ET AL) 6 December 2018 (2018-12-06) * paragraphs [0038], [0039], [0042] * * figure 1 * | 1-4, 7-10, 13-15 | |

The present search report has been drawn up for all claims

| Place of search | Date of completion of the search | Examiner |
|---|---|---|
| Munich | 7 March 2024 | Gärtner, Andreas |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

24

## ANNEX TO THE EUROPEAN SEARCH REPORT
## ON EUROPEAN PATENT APPLICATION NO.

EP 23 20 5911

This annex lists the patent family members relating to the patent documents cited in the above-mentioned European search report.
The members are as contained in the European Patent Office EDP file on
The European Patent Office is in no way liable for these particulars which are merely given for the purpose of information.

07-03-2024

| Patent document cited in search report | | | Publication date | Patent family member(s) | | | Publication date |
|---|---|---|---|---|---|---|---|
| US 2022187509 | A1 | | 16-06-2022 | CN | 113892255 | A | 04-01-2022 |
| | | | | US | 2022187509 | A1 | 16-06-2022 |
| | | | | WO | 2020190602 | A1 | 24-09-2020 |
| US 2015271467 | A1 | | 24-09-2015 | US | 2015271467 | A1 | 24-09-2015 |
| | | | | WO | 2015143347 | A1 | 24-09-2015 |
| US 2022293268 | A1 | | 15-09-2022 | JP | WO2021141048 | A1 | 15-07-2021 |
| | | | | US | 2022293268 | A1 | 15-09-2022 |
| | | | | WO | 2021141048 | A1 | 15-07-2021 |
| US 2019090753 | A1 | | 28-03-2019 | AU | 2017271135 | A1 | 31-01-2019 |
| | | | | EP | 3478182 | A2 | 08-05-2019 |
| | | | | US | 2019090753 | A1 | 28-03-2019 |
| | | | | US | 2024032799 | A1 | 01-02-2024 |
| | | | | WO | 2017203369 | A2 | 30-11-2017 |
| US 2018344145 | A1 | | 06-12-2018 | NONE | | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2013038689 A1

- EP 4000494 A1

**Non-patent literature cited in the description**

- **JIN-BO XU** ; **YOU-RAN ZHAO** ; **RONG-YING YUAN** ; **XIAO-WEI LI** ; **CHAO LIU** ; **QIONG-HUA WANG**. Electrowetting liquid lens integrating adaptive liquid iris. *Optics & Laser Technology*, February 2024, vol. 169, 110023